# EUROPEAN PATENT SPECIFICATION

(11) **EP 2 644 135 B1**
(45) Date of publication and mention of the grant of the patent: **01.08.2018**
(21) Application number: 11843389.5
(22) Date of filing: 15.09.2011
(51) Int. Cl.: A61B 17/32, A61B 17/16

(54) **SURGICAL MILLING CUTTER**
CHIRURGISCHES FRÄS- UND SCHNEIDEWERKZEUG
FRAISE CHIRURGICALE

(30) Priority: 26.11.2010 CN 201010560283
(43) Date of publication of application: 02.10.2013
(73) Proprietor: Chongqing Ruzer Pharmaceutical Co., Ltd., Yubei District Chongqing 401120 (CN)
(72) Inventor: YE, Lei, Chongqing 401120 (CN); ZHOU, Jian, Chongqing 401120 (CN); FENG, Hua, Chongqing 401120 (CN); LI, Fei, Chongqing 401120 (CN); ZHU, Hengyang, Chongqing 401120 (CN); LI, Congxiao, Chongqing 401120 (CN)
(74) Representative: V.O.
(86) International application number: PCT/CN2011/079701
(87) International publication number: WO 2012/068917

(56) References cited:
- CN-A- 101 926 672
- CN-A- 102 059 378
- CN-C- 100 536 796
- CN-U- 201 861 715
- DE-U1-202008 016 969
- US-A- 4 884 569
- US-A1- 2006 122 640

## Description

### Field of the Invention

The invention relates to a surgical cutting instrument and, more particularly, to a surgical milling cutter.

### Description of the Prior art

US 2006/0122640 A1 discloses a surgical milling cutter. During an operation, a milling cutter is used to perform some cutting work.

The milling cutter includes a milling cutter bracket, a locking apparatus, a locking seat and a bottom main machine in top-down sequence. An L-shaped bracket is provided on the top of the milling cutter bracket, a hole is made on the short side of the L-shaped bracket, and the cylindrical bit on the front of the milling cutter bit projects into the hole to ensure a preferred bearing effect for the milling cutter. To protect the milling cutter, the cylindrical bit partially projects into the hole with some of the cylindrical bit left outside of the hole. In prior art, the short side of the L-shaped bracket is designed to be a plane structure. When cutting an object, the cylindrical bit would not function if it contacts the object, which would cause uneven cutting to the object. Additionally, the structure makes it difficult for the milling cutter to turn direction during the cutting process, which makes the operation inconvenient.

The locking apparatus is used to secure the blade of the milling cutter. The conventional securing mode is a rotating mode, the blade is locked or released by rotating to a certain angle, which is inconvenient in use.

The locking seat is connected between the main machine and the milling cutter bracket, which effects to lock the blade during the operation. A connecting shaft structure is provided in the locking seat, the lower end of which is connected to the output shaft of the lower mobile phone power, the upper end of which is connected to the transmission rod of the blade, thus effecting as a bridge therebetween.

The main machine includes the main machine housing and the electric motor in the main machine. The power output by the electric motor drives the blade to rotate by the transmission rod in the locking seat. The main machine is connected to the locking seat with the bolts or the snap-fit connection in prior art. The method of bolt connection is inconvenient, and the snap-fit connection alone is unable to avoid the rotation of the locking seat during the operation of the milling cutter.

### Summary of the Invention

The object of the invention is to provide a surgical milling cutter which prevents the cylindrical bit from contacting the object to be cut, and which also can rotate flexibly and be locked readily, and whose locking seat and main machine are secured readily.

To achieve the object, the following technical scheme is adopted.

A surgical milling cutter includes a milling cutter bracket, a locking apparatus, a locking seat and a bottom main machine connected sequentially and it is characterized in that:
the surgical milling cutter bracket includes a retaining seat with a through hole, a finger guide with an L-shaped bracket on its top is disposed at the upper part of the retaining seat, a downward protrusion is disposed at the end of the short side of the L-shaped bracket, and the lowest point of the protrusion is lower than the lowest point of a cylindrical bit of the milling cutter during normal operation of the milling cutter;
the locking apparatus includes a flange with a through hole, a bearing is disposed at the inner wall of a small cylinder above the flange, a locking sleeve sleeves the flange, a protrusion is disposed on the inner wall at the upper part of the locking sleeve, and a conical surface is formed at the inner wall at the lower part of the locking sleeve; a third spring is disposed between the bottom of the conical surface and the upper bottom of the flange, and the pressing plate is threadedly connected to the outer wall on the top of the small cylinder above the flange and presses the protrusion; a conical hole corresponding to the conical surface is made on the wall of the small cylinder above the flange, and a steel ball is disposed in the conical hole;
the locking seat includes a connecting shaft having an upper connecting bit and a lower connecting bit, a bearing disposed at the inner wall of the bottom of the locking seat and connected to the connecting shaft in an interference fit mode, the lower part of the connecting shaft is provided with a long waist-shaped hole, a pin passes through the long waist-shaped hole, fixing the lower connecting bit with the connecting shaft, and a fourth spring sleeves the connecting shaft between the upper connecting bit and the lower connecting bit;
a groove corresponding to the conical hole is formed on the transmission rod of the milling cutter, and the lower end of the transmission rod is snap-connected to the upper end of the connecting shaft;
the main machine includes a main machine housing and an electric motor, a first protruding cylinder with a through hole at its center is disposed at the upper part of the main machine housing, a first bolt connects a pressing plate and the main machine housing together, a snap-fit notch is provided in the inner wall of the first protruding cylinder, an end with a barb of a snap retainer is disposed in the snap-fit notch, and a bolt provided with a second spring passes through the first protruding cylinder to connect to the other end of the snap retainer; a protrusion is disposed at the inner wall of the locking seat, at least a positioning device is disposed at the main machine housing, the positioning device includes a second protruding cylinder disposed in the through hole of lower cylinder of the first protruding cylinder, a first spring is disposed between the first protruding cylinder and the main machine housing, the upper part of the second protruding cylinder passes the pressing plate; and the bottom of the locking seat is provided with a groove corresponding to the second protruding cylinder.

To allow the transmission rod to bear uniform stress, two bearings are disposed below the outer wall and above the upper bottom.

To ensure the two bearings to be securely fixed, a supporting member is disposed between two bearings.

To facilitate the connection of the reducer, the inner wall of the big cylinder below the flange is provided with the inner threads.

A bolt with a through hole at its center is fixed in the cavity of the finger guide; a pressing plate is disposed at the upper part of the retaining seat, and the nut passes through the hole of the pressing plate to connect to the bolt.

A finger guide is disposed at the lower part of the retaining seat, a T-shaped bolt is fixed in the cavity of the finger guide, a pressing plate is disposed at the upper part of the retaining seat, and a nut passes through the hole of the pressing plate to connect to the bolt.

To ensure the smooth motion of the milling cutter, a bearing is disposed at the upper part of the finger guide.

To avoid the excessively flexibility of the finger guide, a spring sleeves the bolt.

To prevent skidding, the outer surfaces of the retaining seat and finger guide are both provided with anti-slip grooves.

An outer sleeve sleeves the connecting shaft.

The lower end surface of the lower connecting bit is provided with a cross-shaped groove.

As an optimized one, the number of the positioning device is two, and they are apart from each other by 180° to further keep the locking seat stationary.

As an optimized one, to facilitate the installation, the number of the grooves is four, and they are distributed on the same circumference evenly.

The invention adopts the protrusion to prevent the object from contacting the cylindrical bit, thus to avoid cutting the object unevenly. Meanwhile, the finger guide may drive the L-shaped bracket to rotate to change the motion direction of the milling cutter, which facilitates the operation. The lower connecting bit of the connecting shaft is elastically connected to the power output shaft of the mobile phone. Thus, the requirement for the processing precision is low. During the operation, the projecting length of the lower connecting bit of the connecting shaft may be adjusted dynamically, which prolongs the service life of the connecting shaft. The locking seat would not be rotated during the operation of the milling cutter to ensure safety. Furthermore, the locking seat is connected to the main machine housing readily and quickly, which facilitates the use.

### Brief Description of the Drawings

FIG. 1 is a structural diagram of an embodiment of the invention.
FIG. 2 is a structural diagram of the milling cutter bracket in FIG. 1.
FIG. 3 is a structural diagram of the locking apparatus in FIG. 1.
FIG. 4 is a top view of the main machine in FIG. 1.
FIG. 5 is a partially sectional diagram taken along the line A-A in FIG. 4.
FIG. 6 is a partially sectional diagram taken along the line B-B in FIG. 4.
FIG. 7 is a partially sectional diagram taken along the line C in FIG. 5.
FIG. 8 is a partially sectional diagram taken along the line D in FIG. 6.
FIG. 9 is a top view of FIG. 3.

### Detailed Description of the Preferred Embodiments

The invention is described in detail with the appended drawings and the embodiment.

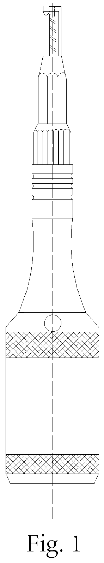

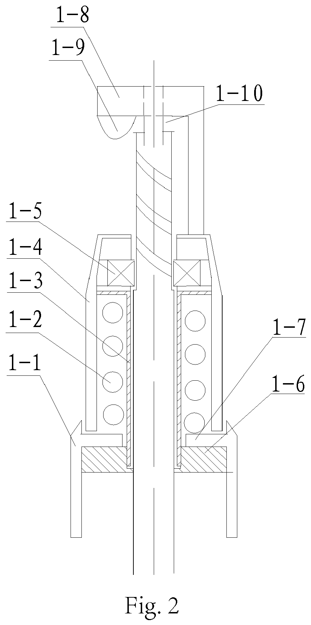

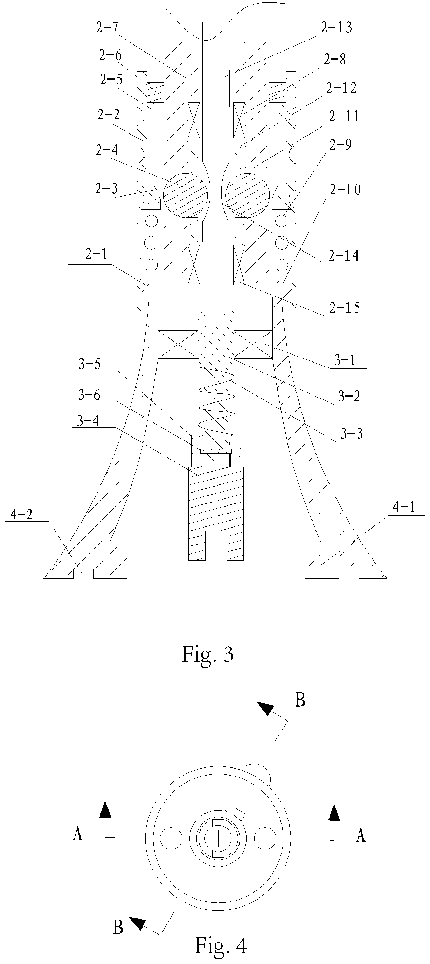

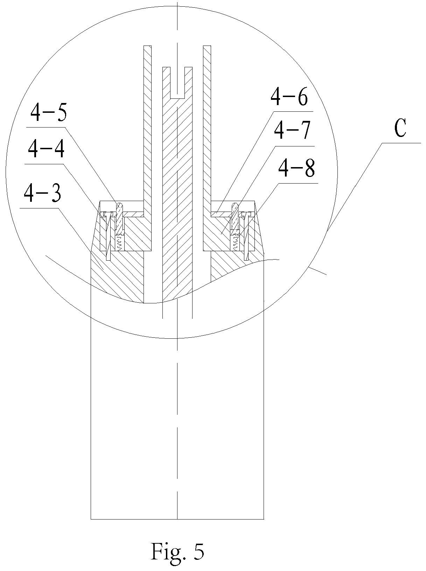

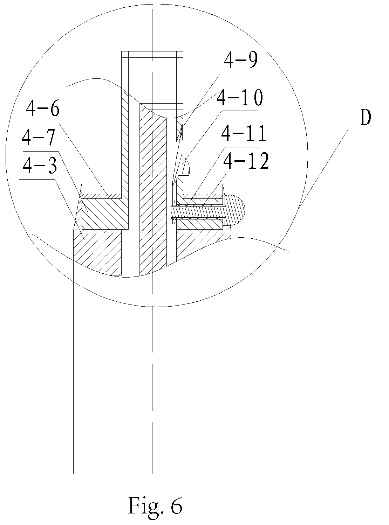

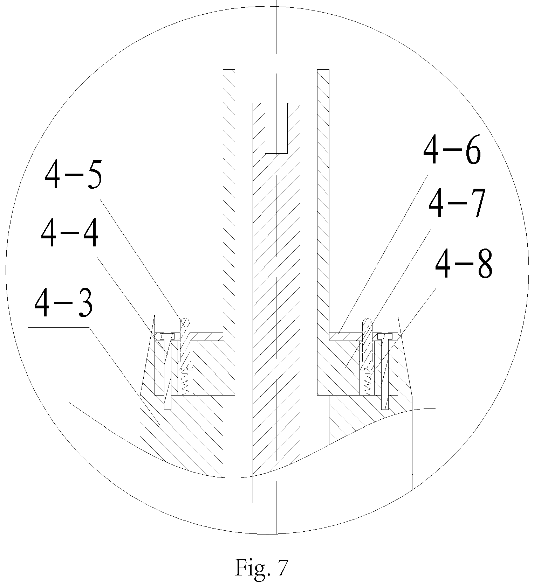

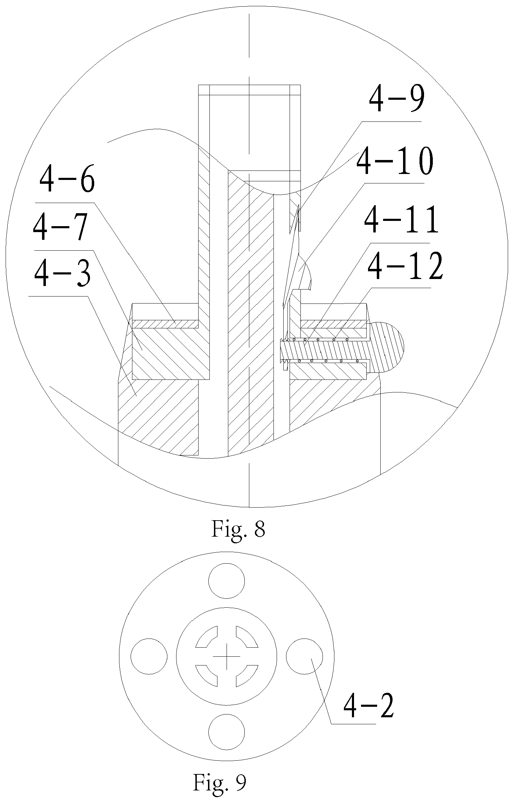

As shown in FIG. 1 to FIG. 9, a surgical milling cutter includes a milling cutter bracket, a locking apparatus, a locking seat and a bottom main machine that are successively connected. The surgical milling cutter bracket includes a retaining seat 1-1 with a through hole. A finger guide 1-4 with an L-shaped bracket on its top is disposed at the upper part of the retaining seat 1-1. A downward protrusion 1-9 is disposed at the end of the short side 1-8 of the L-shaped bracket, and the lowest point of the protrusion 1-9 is lower than the lowest point of the cylindrical bit 1-10 during the normal operation of the milling cutter. Thus, it is impossible for the object to contact the cylindrical bit, and thus to avoid uneven cutting of the object. A T-shaped bolt 1-3 with a through hole is disposed in the cavity of the finger guide 1-4. A pressing plate 1-7 is disposed at the upper part of the retaining seat 1-1, and a nut 1-6 passes through the hole of the pressing plate 1-7 to connect to the bolt 1-3.

A bearing 1-5 is disposed at the upper part of the finger guide 1-4. A spring 1-2 sleeves the bolt 1-3. The outer surfaces of the retaining seat 1-1 and finger guide 1-4 are both provided with anti-slip grooves. The bearing 1-5 provides the milling cutter with an adjacent force point to ensure the smooth motion of the milling cutter. The spring 1-2 ensures that a certain force exists between the finger guide 1-4 and the retaining seat, thus to avoid misoperation caused by the excessively flexible rotation of the finger guide 1-4.

When the finger guide 1-4 is rotated, the bolt 1-3 and the nut 1-6 are rotated therewith. Thus, the finger guide 1-4 drives the L-shaped bracket to rotate to change the motion direction of the milling cutter to facilitate the operation.

The locking apparatus includes a flange 2-1, and a bearing 2-8 is disposed at the inner wall of the small cylinder above the flange. Two bearings 2-8 are disposed below the outer wall 2-7 and above the upper bottom 2-10, respectively. A supporting member 2-12 is disposed between the two bearings 2-8. The two bearings may effectively ensure the smooth motion of the transmission rod to reduce the generated heat. A pressing piece 2-15 is threadedly connected to the upper bottom 2-10 to press against the bearing 2-8 and the supporting member 2-12 to secure the bearing 2-8 and prevent it from falling off. A locking sleeve 2-2 sleeves the flange. A protrusion 2-5 is disposed on the inner wall at the upper part of the locking sleeve 2-2, and a conical surface 2-3 is formed at the inner wall at the lower part of the locking sleeve 2-2. A third spring 2-9 is disposed between the bottom of the conical surface 2-3 and the upper bottom 2-10 of the flange. The pressing plate 2-6 is threadedly connected to the outer wall 2-7 on the top of the small cylinder above the flange and presses the protrusion 2-5. A conical hole 2-11 communicating with the through hole of the flange is made on the wall of the small cylinder above the flange. The steel ball 2-4 is disposed in the conical hole 2-11, and a groove 2-14 corresponding to the conical hole 2-11 is formed on the transmission rod 2-13 of the milling cutter. The inner wall of the big cylinder below the flange is provided with the inner threads. In this embodiment, the number of the conical holes 2-11 and the steel balls 2-4 are two. As an equivalent modification, the number also may be three, thus to obtain a better locking effect. By adopting this locking structure, when the transmission rod needs to be inserted, the locking sleeve just need to the pressed. Then the conical surface moves down, and the steel balls rolls outwards. After the transmission rod reaches the designated position, the locking sleeve is released. Under the action of the spring, the locking sleeve is raised, and conical surface presses against the steel ball to move inward, thus to be fastened with the snap-fit groove of the transmission rod to secure the transmission rod. The locking or releasing process is easy and convenient in this locking mode, and the elements are securely fixed, which offers an improved safety.

The locking seat includes a bearing 3-1 disposed at the inner wall of the bottom of the locking seat, and a connecting shaft having an upper connecting bit 3-2 and a lower connecting bit 3-4 is connected to the bearing 3-1 in an interference fit mode. The lower part of the connecting shaft has a long waist-shaped hole, and a pin 3-6 passes through the long waist-shaped hole, fixing the lower connecting bit 3-4 with the connecting shaft. The fourth spring 3-3 sleeves the connecting shaft between upper connecting bit 3-2 and the lower connecting bit 3-4.

An outer sleeve 3-5 sleeves the connecting shaft. One end of the outer sleeve 3-5 is against the fourth spring 3-3, and the other end is against the end surface of the lower connecting bit 3-4. Thus, the lower connecting bit 3-4 is forced evenly and steadily. The lower end surface of the lower connecting bit is provided with a cross-shaped groove, and the top end of the power output shaft of the mobile phone is provided with a line-shaped protrusion. Thus, the above structure facilitates the connection therebetween. When connected, the lower connecting bit 3-4 is pressed by the top end of the power output shaft of the mobile phone, and the fourth spring 3-3 is deformed. The lower connecting bit 3-4 may be adjusted up and down in a small range in the waist-shaped hole which has a rectangle section. This structure avoids the direct rigid connection between the connecting shaft and the power output shaft of the mobile phone, thus to reduce the requirement for the processing precision and also to prolong the service life of the apparatus.

The structure for connecting the locking seat of the milling cutter and the main machine housing is described as follows. A first protruding cylinder 4-7 with a through hole at the center is disposed at the upper part of the main machine housing 4-3. A first bolt 4-4 connects a pressing plate 4-5 and the main machine housing 4-3 together. A snap-fit notch 4-9 is provided in the inner wall of the first protruding cylinder 4-7. An end with the barb 4-10 of the snap retainer is disposed in the snap-fit notch 4-9, and a second bolt 4-11 provided with a second spring 4-12 passes through the first protruding cylinder 4-7 to connect to the other end of the snap retainer. A protrusion 4-1 is disposed at the inner wall of the locking seat. At least a positioning device is disposed at the main machine housing 4-3, and the positioning device includes a second protruding cylinder 4-5 disposed in the through hole of the lower cylinder of the first protruding cylinder 4-7. A first spring 4-8 is disposed between the first protruding cylinder 4-7 and the main machine housing 4-3. The second protruding cylinder 4-5 passes through the hole in the pressing plate 4-6 which has a hole diameter smaller than the radius of the lower cylinder of the first protruding cylinder 4-3. The bottom of the locking seat is provided with a groove 4-2 corresponding to the second protruding cylinder. There are two positioning devices apart from each other by 180°. There are four grooves 4-2 distributing on the same circumference evenly.

In use, the locking seat only needs to be connected to the upper part of the main machine housing 4-3 and rotated slightly. When the second protruding cylinder 4-5 on the locking seat aligns with the groove 4-2 and is pressed tightly, the snap retainer snaps to the protrusion 4-1, and thus the locking seat and main machine housing are connected together tightly. The second protruding cylinder 4-5 has positioning function, which prevents the locking seat from rotating in use. The four grooves 4-2 allow the locking seat to be stalled only by rotating by 90° at the most, which is convenient is use. Two positioning devices ensure the bearing effect additionally, which maintains the rotation of the locking seat better.

The above-mentioned is merely embodiment of the invention and not to be construed in a limiting sense.

## Claims

1. A surgical milling cutter, comprising a milling cutter bracket, a locking apparatus, a locking seat and a bottom main machine that are successively connected, wherein the milling cutter bracket comprises a retaining seat (1-1) with a through hole, a finger guide (1-4) with an L-shaped bracket on top thereof is disposed above the upper part of the retaining seat (1-1), a protrusion (1-9) extends downwardly from the end of the short side (1-8) of the L-shaped bracket, and the lowest point of the protrusion (1-9) is lower than the lowest point of a cylindrical bit (1-10) of the milling cutter during the normal operation of the milling cutter;
a bolt (1-3) with a center through hole is fixed in a cavity of the finger guide (1-4); a pressing plate (1-7) is disposed at the upper part of the retaining seat (1-1), and a nut (1-6) passes through the hole of the pressing plate (1-7) to connect to the bolt (1-3);
the locking apparatus comprises a flange (2-1) with a through hole, a first bearing (2-8) is disposed at the inner wall of a small cylinder above the flange, a locking sleeve (2-2) sleeves the flange, a protrusion (2-5) is disposed on the inner wall at the upper part of the locking sleeve (2-2), and a conical surface (2-3) is formed at the inner wall at the lower part of the locking sleeve (2-2); a third spring (2-9) is disposed between the bottom of the conical surface (2-3) and the upper bottom (2-10) of the flange, and a pressing plate (2-6) is threadedly connected to the outer wall (2-7) on the top of the small cylinder above the flange and presses the protrusion (2-5); a conical hole (2-11) corresponding to the conical surface (2-3) is made on the wall of the small cylinder above the flange, and a steel ball (2-4) is disposed in the conical hole (2-11);
the locking seat comprises a connecting shaft having an upper connecting bit (3-2) and a lower connecting bit (3-4), a second bearing (3-1) disposed at the inner wall of the bottom of the locking seat and connected to the connecting shaft in an interference fit mode, the lower part of the connecting shaft is provided with a long waist-shaped hole, a pin (3-6) passes through the long waist-shaped hole to fix the lower connecting bit (3-4) with the connecting shaft, and a fourth spring (3-3) sleeves the connecting shaft between upper connecting bit (3-2) and the lower connecting bit (3-4);
a groove (2-14) corresponding to the conical hole (2-11) is formed on the transmission rod (2-13) of the milling cutter, and the lower end of the transmission rod (2-13) is snap-connected to the upper end of the connecting shaft;
the main machine comprises a main machine housing (4-3) and an electric motor, a first protruding cylinder (4-7) with a through hole at its center is disposed at the upper part of the main machine housing (4-3), a first bolt (4-4) connects a pressing plate (4-6) and the main machine housing (4-3) together, a snap-fit notch (4-9) is provided in the inner wall of the first protruding cylinder (4-7), an end with a barb (4-10) of a snap retainer is disposed in the snap-fit notch (4-9), and a second bolt (4-11) provided with a second spring (4-12) passes through the first protruding cylinder (4-7) to connect to the other end of the snap retainer; a protrusion (4-1) is disposed at the inner wall of the locking seat; two positioning devices are disposed at the main machine housing (4-3), the positioning device comprises a second protruding cylinder (4-5) disposed in a through hole of the lower cylinder of the first protruding cylinder (4-7), a first spring (4-8) is disposed between the first protruding cylinder (4-7) and the main machine housing (4-3), and the upper part of the second protruding cylinder (4-5) passes through the pressing plate (4-6); the bottom of the locking seat is provided with a groove (4-2) corresponding to the second protruding cylinder (4-5).

2. The surgical milling cutter according to claim 1, **characterized in that** a third bearing (1-5) is disposed at the upper part of the finger guide (1-4).

3. The surgical milling cutter according to claim 2, **characterized in that** a spring (1-2) sleeves the bolt (1-3).

4. The surgical milling cutter according to claim 3, **characterized in that** the outer surfaces of the retaining seat (1-1) and finger guide (1-4) are both provided with anti-slipslip grooves.

5. The surgical milling cutter according to any of claims 1 to 4, **characterized in that** the number of the first bearings (2-8) is two, and they are disposed below the outer wall (2-7) and above the upper bottom (2-10), respectively.

6. The surgical milling cutter according to claim 5, **characterized in that** a supporting member (2-12) is disposed between the two first bearings (2-8).

7. The surgical milling cutter according to claim 6, **characterized in that** the inner wall of the big cylinder below the flange (2-1) is provided with internal threads.

8. The surgical milling cutter according to claim 7, **characterized in that** an outer sleeve (3-5) sleeves the connecting shaft.

9. The surgical milling cutter according to claim 8, **characterized in that** the lower end surface of the lower connecting bit is provided with a cross-shaped groove.

10. The surgical milling cutter according to claim 9, **characterized in that** the number of the positioning device is two, and they are apart from each other by 180°.

11. The surgical milling cutter according to claim 10, **characterized in that** the number of the grooves (4-2) is four, and they are distributed on the same circumference evenly.

## Patentansprüche

1. Chirurgisches Fräs- und Schneidewerkzeug, umfassend eine Fräs- und Schneideklammer, eine Klemmvorrichtung, einen Klemmsitz und eine untere Hauptmaschine, die nacheinander verbunden sind, wobei
die Fräs- und Schneideklammer einen Haltesitz (1-1) umfasst, mit einer Durchgangsbohrung, einer Fingerführung (1-4) mit darauf einer L-förmigen Klammer, die über dem oberen Teil des Haltesitzes (1-1) angeordnet ist, wobei ein Vorsprung (1-9) abwärts von dem Ende der kurzen Seite (1-8) der L-förmigen Klammer verläuft, und der tiefste Punkt des Vorsprungs (1-9) tiefer ist als der tiefste Punkt eines zylindrischen Stücks (1-10) des Fräs- und Schneidewerkzeugs während des normalen Betriebs des Fräs- und Schneidewerkzeugs; wobei
ein Bolzen (1-3) mit einer mittigen Durchgangsbohrung in einem Hohlraum der Fingerführung (1-4) befestigt ist; eine Pressplatte (1-7) an dem oberen Teil des Haltesitzes (1-1) angeordnet ist, und eine Mutter (1-6) durch die Bohrung der Pressplatte (1-7) geht, um mit dem Bolzen (1-3) verbunden zu werden; wobei
die Klemmvorrichtung einen Flansch (2-1) mit einer Durchgangsbohrung umfasst, ein erstes Lager (2-8) an der Innenwand eines kleinen Zylinders über dem Flansch angeordnet ist, eine Klemmbuchse (2-2) den Flansch klemmt, ein Vorsprung (2-5) an der Innenwand am oberen Teil der Klemmbuchse (2-2) angeordnet ist und eine konische Oberfläche (2-3) an der Innenwand des unteren Teils der Klemmbuchse (2-2) gebildet ist; eine dritte Feder (2-9) zwischen dem Boden der konischen Oberfläche (2-3) und dem oberen Boden (2-10) des Flansches angeordnet ist und eine Pressplatte (2-6) eingeschraubt mit der Außenwand (2-7) oben auf dem kleinen Zylinder über dem Flansch verbunden ist und den Vorsprung (2-5) presst; eine konische Bohrung (2-11) entsprechend der konischen Oberfläche (2-3) an der Wand des kleinen Zylinders über dem Flansch gemacht ist und eine Stahlkugel (2-4) in der konischen Bohrung (2-11) angeordnet ist;
der Klemmsitz eine Verbindungswelle mit einem oberen Verbindungsstück (3-2) und einem unteren Verbindungsstück (3-4) umfasst, ein zweites Lager (3-1), angeordnet an der Innenwand des Bodens des Klemmsitzes und verbunden mit der Verbindungswelle in einem Presspassungsmodus, der untere Teil der Verbindungswelle mit einer langen, taillenförmigen Bohrung versehen ist, ein Stift (3-6) durch die lange, taillenförmige Bohrung geht, um das untere Verbindungsstück (3-4) mit der Verbindungswelle zu befestigen, und eine vierte Feder (3-3) die Verbindungswelle zwischen dem oberen Verbindungsstück (3-2) und dem unteren Verbindungsstück (3-4) klemmt;
eine Rille (2-14) entsprechend der konischen Bohrung (2-11) auf der Übertragungsstange (2-13) des Fräs- und Schneidewerkzeugs gebildet ist und das untere Ende der Übertragungsstange (2-13) am oberen Ende der Verbindungswelle eingeschnappt ist; wobei
die Hauptmaschine ein Hauptmaschinengehäuse (4-3) und einen Elektromotor umfasst, ein erster vorspringender Zylinder (4-7) mit einer Durchgangsbohrung an seiner Mitte an dem oberen Teil des Hauptmaschinengehäuses (4-3) angeordnet ist, ein erster Bolzen (4-4) eine Pressplatte (4-6) und das Hauptmaschinengehäuse (4-3) miteinander verbindet, eine Einschnappkerbe (4-9) in der Innenwand des ersten vorspringenden Zylinders (4-7) bereitgestellt ist, ein Ende mit einem Widerhaken (4-10) eines Federhalters in der Einschnappkerbe (4-9) angeordnet ist und ein zweiter Bolzen (4-11), versehen mit einer zweiten Feder (4-12) durch den ersten vorspringenden Zylinder (4-7) geht, um mit dem anderen Ende des Schnapphalters verbunden zu werden; ein Vorsprung (4-1) an der Innenwand des Klemmsitzes angeordnet ist; wobei
zwei Positioniervorrichtungen an dem Hauptmaschinengehäuse (4-3) angeordnet sind, die Positioniervorrichtung einen zweiten vorspringenden Zylinder (4-5) in einer Durchgangsbohrung des unteren Zylinders des ersten vorspringenden Zylinders (4-7) angeordnet ist, eine erste Feder (4-8) zwischen dem ersten vorspringenden Zylinder (4-7) und dem Hauptmaschinengehäuse (4-3) angeordnet ist und der obere Teil des zweiten vorspringenden Zylinders (4-5) durch die Pressplatte (4-6) geht; der Boden des Klemmsitzes mit einer Rille (4-2) entsprechend dem zweiten vorspringenden Zylinder (4-5) versehen ist.

2. Chirurgisches Fräs- und Schneidewerkzeug nach Anspruch 1, **dadurch gekennzeichnet, dass** ein drittes Lager (1-5) an dem oberen Teil der Fingerführung (1-4) angeordnet ist.

3. Chirurgisches Fräs- und Schneidewerkzeug nach Anspruch 2, **dadurch gekennzeichnet, dass** eine Feder (1-2) den Bolzen (1-3) klemmt.

4. Chirurgisches Fräs- und Schneidewerkzeug nach Anspruch 3, **dadurch gekennzeichnet, dass** die äußeren Oberflächen des Haltesitzes (1-1) und der Fingerführung (1-4) beide mit rutschfesten Rillen versehen sind.

5. Chirurgisches Fräs- und Schneidewerkzeug nach einem der Ansprüche 1 bis 4, **dadurch gekennzeichnet, dass** die Anzahl der ersten Lager (2-8) zwei ist und sie jeweils unter der Außenwand (2-7) und über dem oberen Boden (2-10) angeordnet sind.

6. Chirurgisches Fräs- und Schneidewerkzeug nach Anspruch 5, **dadurch gekennzeichnet, dass** ein Stützelement (2-12) zwischen den zwei ersten Lagern (2-8) angeordnet ist.

7. Chirurgisches Fräs- und Schneidewerkzeug nach Anspruch 6, **dadurch gekennzeichnet, dass** die Innenwand des großen Zylinders unter dem Flansch (2-1) mit Innengewinden versehen ist.

8. Chirurgisches Fräs- und Schneidewerkzeug nach Anspruch 7, **dadurch gekennzeichnet, dass** eine äußere Buchse (3-5) die Verbindungswelle klemmt.

9. Chirurgisches Fräs- und Schneidewerkzeug nach Anspruch 8, **dadurch gekennzeichnet, dass** die untere Endoberfläche des unteren Verbindungsstücks mit einer kreuzförmigen Rille versehen ist.

10. Chirurgisches Fräs- und Schneidewerkzeug nach Anspruch 9, **dadurch gekennzeichnet, dass** die Anzahl der Positioniervorrichtung zwei ist und sie 180° Grad auseinander sind.

11. Chirurgisches Fräs- und Schneidewerkzeug nach Anspruch 10, **dadurch gekennzeichnet, dass** die Anzahl der Rillen (4-2) vier ist und sie gleichmäßig auf demselben Umfang verteilt sind.

## Revendications

1. Fraise chirurgicale comprenant un support de fraise, un appareil de verrouillage, un siège de verrouillage et une machine principale inférieure qui sont successivement raccordés, dans laquelle :
le support de fraise comprend un siège de retenue (1-1) avec un trou débouchant, un guide de doigt (1-4) avec un support en forme de L sur sa partie supérieure est disposé au-dessus de la partie supérieure du siège de retenue (1-1), une saillie (1-9) s'étend vers le bas à partir de l'extrémité du côté court (1-8) du support en forme de L, et le point le plus bas de la saillie (1-9) est plus bas que le point le plus bas d'un foret cylindrique (1-10) de la fraise pendant le fonctionnement normal de la fraise ;
un boulon (1-3) avec un trou débouchant central est fixé dans une cavité du guide de doigt (1-4) ; une plaque de pression (1-7) est disposée au niveau de la partie supérieure du siège de retenue (1-1), et un écrou (1-6) passe à travers le trou de la plaque de pression (1-7) pour se raccorder au boulon (1-3) ;
l'appareil de verrouillage comprend une bride (2-1) avec un trou débouchant, un premier palier (2-8) est disposé au niveau de la paroi interne d'un petit cylindre au-dessus de la bride, un manchon de verrouillage (2-2) met en prise par manchonnage la bride, une saillie (2-5) est disposée sur la paroi interne au niveau de la partie supérieure du manchon de verrouillage (2-2), et une surface conique (2-3) est formée au niveau de la paroi interne, au niveau de la partie inférieure du manchon de verrouillage (2-2) ; un troisième ressort (2-9) est disposé entre le fond de la surface conique (2-3) et le fond supérieur (2-10) de la bride, et une plaque de pression (2-6) est raccordée, par filetage, à la paroi externe (2-7) sur la partie supérieure du petit cylindre au-dessus de la bride et comprime la saillie (2-5) ; un trou conique (2-11) correspondant à la surface conique (2-3) est réalisé sur la paroi du petit cylindre au-dessus de la bride, et une bille en acier (2-4) est disposée dans le trou conique (2-11) ;
le siège de verrouillage comprend un arbre de raccordement ayant un foret de raccordement supérieur (3-2) et un foret de raccordement inférieur (3-4) ; un deuxième palier (3-1) disposé au niveau de la paroi interne du fond du siège de verrouillage et raccordé à l'arbre de raccordement par ajustement par serrage, la partie inférieure de l'arbre de raccordement est prévue avec un trou long en forme de resserrement, une broche (3-6) passe à travers le trou long en forme de resserrement pour fixer le foret de raccordement inférieur (3-4) avec l'arbre de raccordement, et un quatrième ressort (3-3) met en prise par manchonnage l'arbre de raccordement entre le foret de raccordement supérieur (3-2) et le foret de raccordement inférieur (3-4) ;
une rainure (2-14) correspondant au trou conique (2-11) est formée sur la tige de transmission (2-13) de la fraise, et l'extrémité inférieure de la tige de transmission (2-13) est encliquetée sur l'extrémité supérieure de l'arbre de raccordement ;
la machine principale comprend un boîtier de machine principale (4-3) et un moteur électrique, un premier cylindre en saillie (4-7) avec un trou débouchant au niveau de son centre, est disposé, au niveau de la partie supérieure du boîtier de machine principale (4-3), un premier boulon (4-4) raccorde une plaque de pression (4-6) et le boîtier de machine principale (4-3) ensemble, une encoche d'encliquetage (4-9) est prévue dans la paroi interne du premier cylindre en saillie (4-7), une extrémité avec un ardillon (4-10) d'un dispositif de retenue d'encliquetage est disposée dans l'encoche d'encliquetage (4-9), et un second boulon (4-11) prévu avec un deuxième ressort (4-12) passe à travers le premier cylindre en saillie (4-7) pour raccorder l'autre extrémité du dispositif de retenue d'encliquetage ; une saillie (4-1) est disposée au niveau de la paroi interne du siège de verrouillage ; deux dispositifs de positionnement sont disposés au niveau du boîtier de machine principale (4-3), le dispositif de positionnement comprend un second cylindre en saillie (4-5) disposé dans un trou débouchant du cylindre inférieur du premier cylindre en saillie (4-7), un premier ressort (4-8) est disposé entre le premier cylindre en saillie (4-7) et le boîtier de machine principale (4-3) et la partie supérieure du second cylindre en saillie (4-5) passe à travers la plaque de pression (4-6) ; le fond du siège de verrouillage est prévu avec une rainure (4-2) correspondant au second cylindre en saillie (4-5).

2. Fraise chirurgicale selon la revendication 1, **caractérisée en ce qu'**un troisième palier (1-5) est disposé au niveau de la partie supérieure du guide de doigt (1-4).

3. Fraise chirurgicale selon la revendication 2, **caractérisée en ce qu'**un ressort (1-2) met en prise par manchonnage le boulon (1-3).

4. Fraise chirurgicale selon la revendication 3, **caractérisée en ce que** les surfaces externes du siège de retenue (1-1) et le guide de doigt (1-4) sont tous deux prévus avec des rainures antiglisse.

5. Fraise chirurgicale selon l'une quelconque des revendications 1 à 4,
**caractérisée en ce que** le nombre de premiers paliers (2-8) est de deux, et ils sont disposés au-dessous de la paroi externe (2-7) et au-dessus du fond supérieur (2-10), respectivement.

6. Fraise chirurgicale selon la revendication 5, **caractérisée en ce qu'**un élément de support (2-12) est disposé entre les deux premiers paliers (2-8).

7. Fraise chirurgicale selon la revendication 6, **caractérisée en ce que** la paroi interne du grand cylindre au-dessous de la bride (2-1) est prévue avec des filetages internes.

8. Fraise chirurgicale selon la revendication 7, **caractérisée en ce qu'**un manchon externe (3-5) met en prise par manchonnage l'arbre de raccordement.

9. Fraise chirurgicale selon la revendication 8, **caractérisée en ce que** la surface d'extrémité inférieure du foret de raccordement inférieur est prévue avec une rainure de forme transversale.

10. Fraise chirurgicale selon la revendication 9, **caractérisée en ce que** le nombre de dispositifs de positionnement est de deux, et ils sont éloignés l'un de l'autre de 180°.

11. Fraise chirurgicale selon la revendication 10, **caractérisée en ce que** le nombre de rainures (4-2) est de quatre, et elles sont réparties régulièrement sur la même circonférence.
